# EUROPEAN PATENT APPLICATION

(11) **EP 4 821 515 A1**
(43) Date of publication of application: **14.10.2026**
(21) Application number: 26170009.0
(22) Date of filing: 31.03.2026
(51) Int. Cl.: A61L 26/00

(54) **FLOWABLE SEALANT HEMOSTAT**

(30) Priority: 09.04.2025 WO PCT/CN2025/087907
(71) Applicant: Guangzhou Bioseal Biotech Co., Ltd., Guangzhou, Guangdong 510530 (CN)
(72) Inventor: DENG, XUEQIN, Guangzhou, 510530 (CN); WAN, XIANG, Shanghai, 200233 (CN); WANG, YAGUO, Guangzhou, 510530 (CN); WANG, YALIN, Guangzhou, 510530 (CN); WANG, YAPING, Guangzhou, 510530 (CN); YANG, YANG, Guangzhou, 510530 (CN)
(74) Representative: Carpmaels & Ransford LLP

(57) **Abstract**

Disclosed herein are two-phase flowable compositions in the form of a paste comprised of (i) a powder of fibrinogen, fibrin, and thrombin; and (ii) a dispersant of glycerol and an aqueous solution. Processes and kits for preparing the compositions are also disclosed. Uses of the compositions for treating bleeding in a subject are also disclosed.

## Description

### FIELD OF THE INVENTION

The present invention relates, inter alia, to flowable hemostatic compositions comprising a blend of fibrinogen, thrombin, and a dispersant.

### BACKGROUND OF THE INVENTION

In various situations during surgery therapeutic agents are spread inside the human body, for prevention of bleeding after the surgery. Existing agents may have some drawbacks, for example, sheets are difficult to place in minimal invasive surgeries (MIS).

The selection of appropriate methods or products for the control of bleeding is dependent upon many factors, which include, but are not limited to, bleeding severity; anatomical location of the source and the proximity of adjacent critical structures, whether the bleeding is from a discrete source or from a broader surface area; visibility and precise identification of the bleeding source; and access to the source.

In an effort to address the above-described problems, materials have been developed for controlling excessive bleeding. Topical Absorbable Hemostats (TAHs) are widely used in surgical applications. TAHs encompass products based on oxidized cellulose (OC), gelatin, collagen, chitin, chitosan, etc. To improve the hemostatic performance, scaffolds based on the above materials can be combined with biologically-derived clotting factors, such as thrombin and fibrinogen.

However, difficult-to-access bleeding sites, including deep anatomic sites, tight crevice, or cavity gap are important instances in Surgeon's operation. There is a great need for developing hemostats which could reach precisely into the tight and deep bleeding sites. It is desired that the hemostat will exhibit flowability without compromising adherence, that is, the hemostatic must adhere strongly to the tissue of choice even under high fluid pressures, wet and dynamic conditions and stay in place until hemostasis is achieved.

PCT Patent Application No. PCT/CN2021/087167 discloses two-component flowable compositions comprised of: (i) a powder of: oxidized cellulose, fibrinogen and thrombin, with the weight ratio of the oxidized cellulose to the fibrinogen ranging from about 1:5 to about 2:1, respectively; and (ii) a dispersant comprised of glycerol and an aqueous solution, and with the composition being in the form of a pasty suspension at a room temperature. Also disclosed are uses of the compositions for treating a bleeding tissue, and methods for making the compositions.

### SUMMARY OF THE INVENTION

The present invention relates, *inter alia,* to flowable hemostatic compositions comprising a blend of fibrinogen, thrombin, and a dispersant.

An object of the present invention is to provide a composition for preparing a spreadable hemostat which can easily be applied to bleeding sites of need, including in difficult-to-reach areas of the body, can adhere to the bleeding sites quickly and then form a sealant therein. A specific object of this invention is to develop a flowable hemostat with both flowability and adherence to tissue and/or platelets after applying it into bleeding sites during surgery. The inaccessible bleeding sites, including deep anatomic sites, tight crevice, or cavity gaps, are visible but difficult to access by traditional medical device, and are important instances in surgeon's operation. It is in great need to develop the hemostats which could reach precisely and flow flexibly into the tight and deep bleeding sites, with the flowability being without compromising adherence. Platelet adherence is critical for hemostat which should reach precisely into the bleeding sites and stay in place until hemostasis is achieved.

Fibrinogen interacts with thrombin in aqueous media. Finding a suitable formulation having an appropriate consistency is not straight-forward since the commonly used dispersant, such as water or hydrophilic dispersant, cannot be used without compromising the pastiness, adhesiveness, and/or hemostatic functions. Thus, for a stable liquid/flowable composition comprising both fibrinogen and thrombin, non-aqueous medium (dispersant) is commonly used prior to application on bleeding sites e.g., for sealing and hemostasis.

Accordingly, the present invention is based on the surprising finding that such a pasty suspension comprising a powder of fibrinogen/fibrin, thrombin dispersed in a dispersant comprising e.g., glycerol and aqueous solution provides superior hemostatic functionalities. Such a composition may therefore be applied to a site of need in order to obtain biological activity such as controlling bleeding, especially in difficult-to-reach areas of the body. The inventors developed a flowable hemostatic paste which could stay in place after applying it into the bleeding sites. The disclosed paste can absorb part of the flooded blood, which helps the paste to adhere into the bleeding sites quickly and closely into the deep bleeding sites; and can form a protein sealant on the bleeding sites, which helps to further enhance the adherence to the tissues.

The use of aqueous solution in such a suspension is counter-intuitive, and it may be assumed, without being bound by any particular theory or mechanism, that after mixing the porous powder of the invention in the dispersant the uniformly dispersed biologics forms a fibrin, which is at least partially cross-linked in a glycerol/saline system which forms a paste with rich thrombin remaining in the system.

As such, the disclosed composition has been demonstrated to be an easy-to-use, stable and efficacious hemostat.

According to an aspect of the present invention, there is provided a two-phase flowable composition in the form of a paste at a room temperature, comprising:
(i) a powder comprising fibrinogen and fibrin, being present together at an amount of at least about 70% by weight, and thrombin; and
(ii) a dispersant comprising glycerol and an aqueous solution,
wherein:
The dispersant is present at a dispersant-to-powder ratio ranging from about 3:1 to about 5:1 (v/w; ml per g), respectively; and wherein the glycerol is present at a concentration of above about 30% by volume of said dispersant.

In some embodiments, the powder is in the form of porous particles. In some embodiments, at least 50% of the particles are characterized by having a pore size ranging from about 10 to about 30 µm.

In some embodiments, the composition comprises fibrin at an amount of about 60% to 80%, by weight.

In some embodiments, the fibrinogen is present at a concentration range of about 12 to about 20% w/w. In some embodiments, the fibrin is present at a concentration range of about 53 to about 82% w/w. In some embodiments, the thrombin is present at a concentration range of 4 to 25% w/w.

In some embodiments, the fibrin is at least partially cross-linked.

In some embodiments, the fibrinogen and/or fibrin are present in the powder at an amount of about 80% by weight.

In some embodiments, the powder further comprises a calcium salt. In some embodiments, the calcium salt is CaCl₂, being at an amount of up to about 3%, e.g., 0.5 to 3% by weight, e.g., about 2% w/w. Herein "CaCl₂" is also meant to further include any hydrate thereof, for example, and without limitation, CaCl₂·H₂O (hydrate), CaCl₂·2H₂O (dihydrate), or a combination thereof.

In some embodiments, the thrombin is present at an amount of about 2000 to about 4000 IU, typically per 1 gr powder.

In some embodiments, at least 80% of the powder is in the form of particles sized below 500 µm. In some embodiments, at least part of the particles is agglomerated. In some embodiments, the glycerol is present at a concentration ranging from about 30% to about 80%, by weight of the dispersant. In some embodiments, the aqueous solution comprises saline.

In some embodiments, the composition (the paste) is characterized by uniform viscosity. In some embodiments, the paste is characterized by viscosity ranging from about 3000 to about 5000 Pa·s at about 25 °C. In some embodiments, the viscosity varies within less than about 30% after storing at about 25 °C for 24 h. In some embodiments, the composition is characterized by a swelling ratio of at most 10% at about 25 °C upon contact with blood.

In some embodiments, the composition is absent from oxidized cellulose.

In some embodiments, the composition is absent from a buffering agent.

In some embodiments, the composition is a hemostatic composition.

In some embodiments, the composition is for use in a method for treating a bleeding tissue, the method comprising applying said composition to the bleeding tissue.

In some embodiments, the method comprises minimal invasive surgery e.g., laparoscopy.

According to another aspect, there is provided a method of making a hemostatic composition characterized by viscosity ranging from about 3000 to about 5000 Pa·s at about 25 °C and/or a swelling ratio of at most 10% at about 25 °C, the method comprising the following steps performed subsequently:
(i) whipping a fibrinogen solution so as to obtain foam;
(ii) spraying a thrombin solution into the foam, so that the foam further incorporates the thrombin;
(iii) stirring the foam containing the thrombin so as to allow partial conversion of fibrinogen into fibrin;
(iv) lyophilizing the fibrin;
(v) milling the lyophilized fibrin, thereby obtaining a uniform powder, and
(vi) mixing the powder with a dispersant comprising an aqueous solution, glycerol and thrombin, in a condition allowing making the hemostatic composition.

In some embodiments, the condition is selected from one or more from (a) to (c): (a) the dispersant comprises thrombin an amount of about 200 to about 800 IU per 1 ml; (b) the dispersant is added to the powder at a dispersant-to-powder ratio ranging from 3:1 to 5:1 (v/w; ml/g), respectively, and (c) at least about 70% of the foam obtained in step (ii) comprises fibrin.

In some embodiments, the thrombin solution in step (ii) further comprises a calcium salt.

In some embodiments of this aspect, the calcium salt is CaCl₂, being at a concentration of 0.5 to about 3% w/v.

According to another aspect, there is provided a kit comprising:
(a) a container containing a powder comprising, fibrinogen, thrombin, and optionally fibrin, wherein the fibrinogen+fibrin are present at a total amount of at least about 70% by weight; and
(b) a container containing a dispersant comprising glycerol and optionally an aqueous solution, and optionally(c) instructions for use.

Embodiments of the powder are described hereinthroughout and are incorporated to the aspect of the kit as well.

In some embodiments, the kit comprises an applicator device.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is contemplated that the particulars shown are by way of example and for purposes of illustrative discussion of embodiments of the invention. In this regard, the description taken with the drawings makes apparent to those skilled in the art how embodiments of the invention may be practiced.

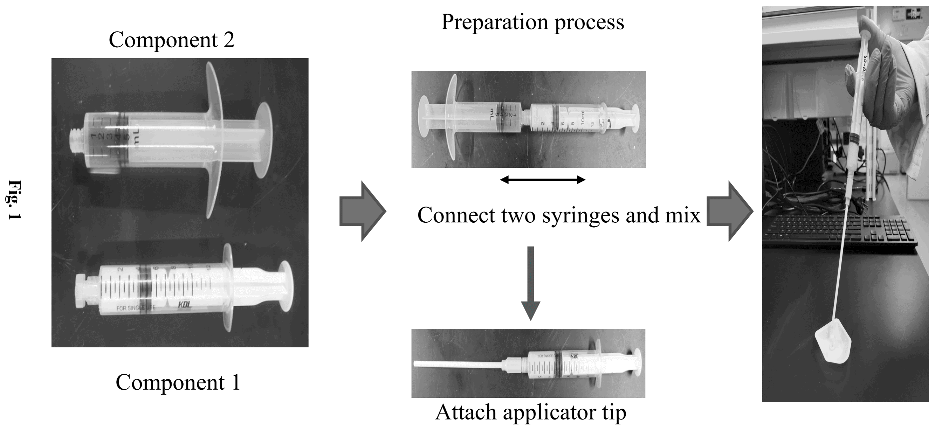

**Figure 1** presents photographic images showing a process of mixing a first component comprising a powder of fibrinogen and thrombin, and a second component containing a dispersant comprising glycerol an aqueous solution using an applicator made of two syringes, according to an embodiment of the present invention, following by applying the obtained paste upon connecting an applicator tip to the applicator. "Component 1" is 1 g fibrin sealant (FS) powder plus 3300 IU potency thrombin powder with a CaCl₂ (about 21 mg). "Component 2" is 4 ml of 50% v/v glycerin solution (in physiological saline).

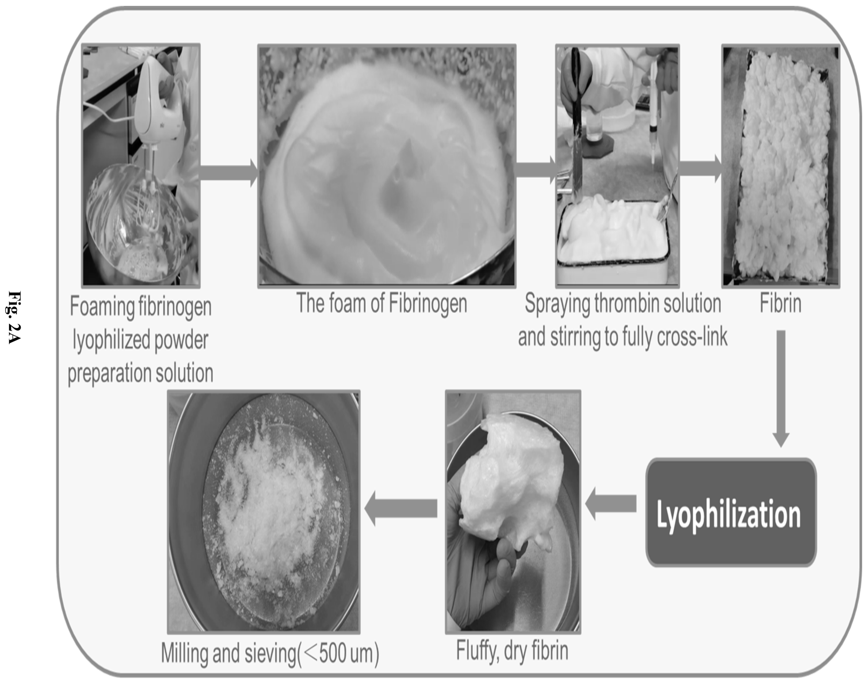

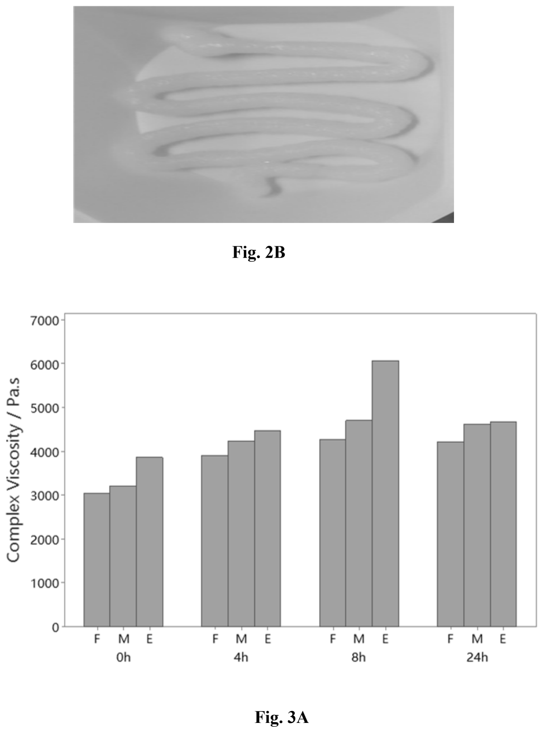

**Figures 2A-2B** present a flowchart with photographic images showing an exemplary embodiment of process making the powder of the invention (Figure 2A), and a photographic image of the obtained paste (Figure 2B).

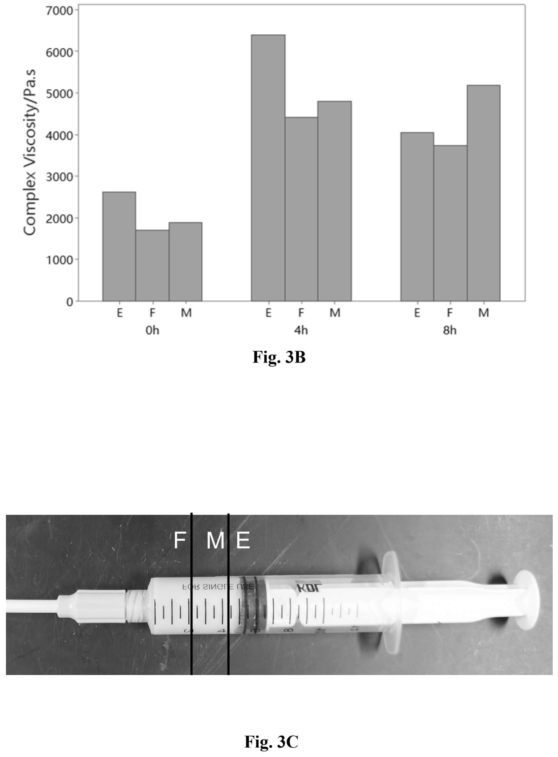

**Figures 3A-3C** present comparative viscosities of a flowable paste of the invention (bar graphs in Figure 3A) versus Oxidized Regenerated Cellulose (ORC) paste (bar graphs in Figure 3B), being stood at room temperature for the indicated time up to 24 hours; to test the homogeneously of the samples were taken from various parts of the syringe: F (Front), M (Middle), E (End) as shown in Figure 3C.

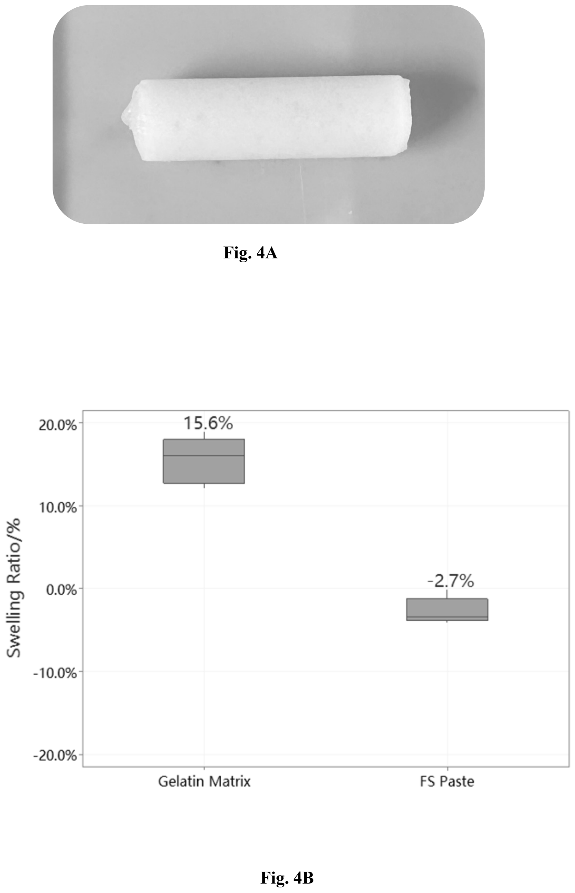

**Figures 4A-4B** present the swelling ratio of a flowable paste of the invention compared to the swelling ratio of a gelatin matrix: samples were first incubated for 24 hours in a Phosphate Buffered Saline (PBS) solution allowing them to swell (Figure 4A) and then the weight increase of each sample was measured. Five sections of a sample ware tested.

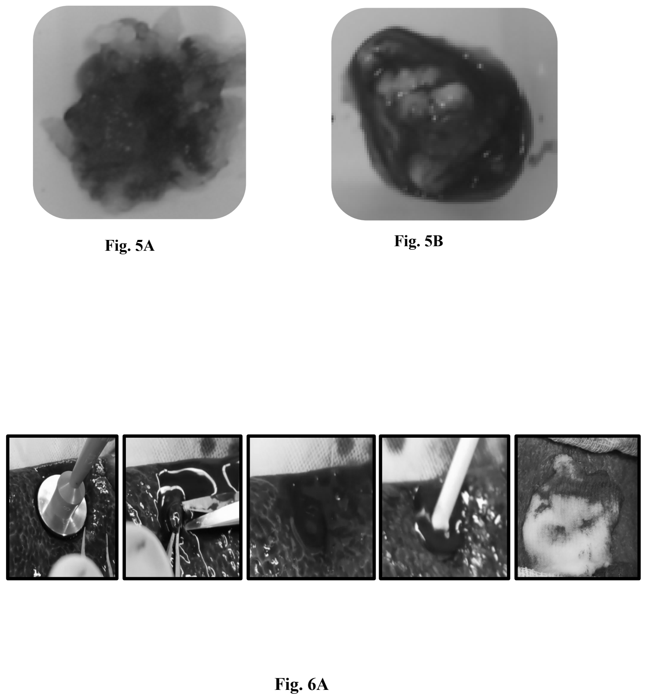

**Figures 5A-5B** present comparative photographic images demonstrating blood absorbing capabilities of: a flowable paste of the invention (Figure 5A) and that of the ORC paste (Figure 5B). It is shown that the flowable paste of the invention easily and uniformly absorbs blood as compared to the ORC paste, thereby accelerating the coagulation cascades to form endogenous fibrin clot.

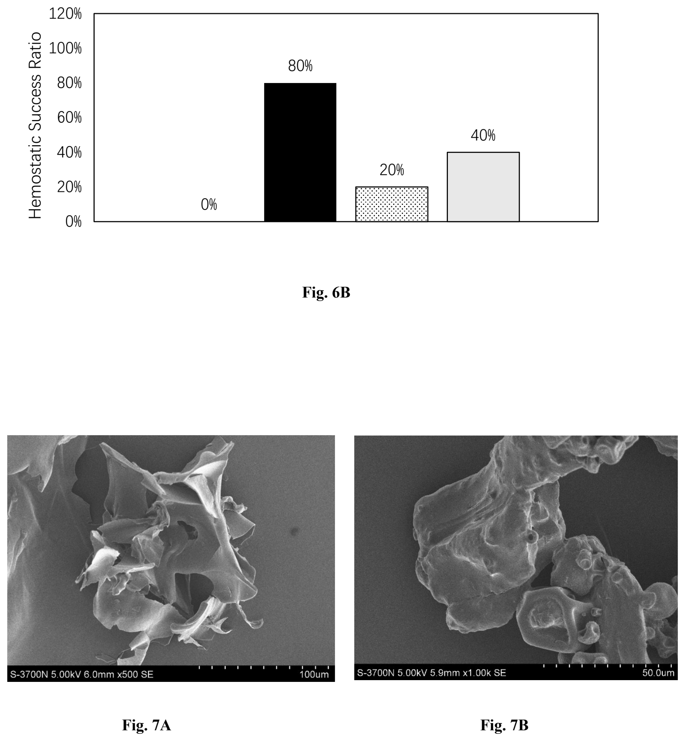

**Figures 6A-6B** present an efficacy test in in-vivo Spleen Punch Model; photographic images (Figure 6A) of the steps from left to right: a wound was made by biopsy punching (6 mm in diameter and 3 mm in depth); the center core (tissue plug) was removed; moderate bleeding was going on; a tested sample was applied to cover the wound site; and after pressing with a wet gauze for 2 minutes an assessment of the hemostatic outcome (success or failure) was made; five samples were tested for paste, and the success rate is shown in a bar graph (Figure 6B; from left to right: negative control - 0%, the fibrin sealant of an embodiment of the invention - 80%; gelatin matrix - 20%, and the ORC paste - 40%).
**Figures 7A-7B** present Scanning Electron Microscopic (SEM) images of the fibrin sealant powder made according to some embodiments of the present invention (Figure 7A; bar is 100 µm) demonstrating a loose porous powder, compared to flowable powder made with ORC (ORC powder) demonstrating non-porous structure (Figure 7B; bar is 50 µm).

### DESCRIPTION OF EMBODIMENTS OF THE INVENTION

The production of flowable hemostat of fibrin composite matrix that is easily prepared and applied for intra-operative use in difficult-to-access bleeding sites is challenging, especially during minimally invasive surgeries (MIS) such as, e.g., endoscopy.

According to the feature of "bleeding sites", the inaccessible sites including deep anatomic sites, tight crevice, or cavity gap, visible but difficult to access by traditional medical device, are important instances in surgeon's operation.

An object of the present invention is therefore to provide an improved composition which may be easily applied to a bleeding site of need, especially in difficult-to-reach areas of the body. A further advantage of the compositions of the invention is that they are bioabsorbable, and therefore may be left behind following surgery without causing any side effects.

By "applied to a bleeding site of need" it is meant to refer, e.g., to a topical application of the composition at, or on/near a bleeding surgical site of a tissue.

According to an aspect of the present disclosure, there is provided a two-phase flowable composition in the form of a paste at around room temperature comprising: (a) a powder comprising: (i) fibrinogen at an amount of at least about 70% by weight; (ii) thrombin; (iii) a calcium salt; and (b) a dispersant comprising glycerol and an aqueous solution. In some embodiments, the dispersant is present at a dispersant-to-powder ratio ranging from about 3:1 to about 5:1 (v/w; ml per g), respectively; in some embodiments, the glycerol is present at a concentration of above about 30% by volume of the dispersant.

The terms "liquid medium", "medium", or "dispersant" may be used hereinthroughout interchangeably. Herein, the term "dispersant" is meant to refer to a liquid medium, optionally a liquid medium having a viscosity having, or is capable of providing, a pasty consistency. Thus, the term "dispersant" is used in a generic meaning, and may be employed rather than the word "solvent" only to clarify that the disclosed blend or powder comprised of e.g., fibrinogen or thrombin is substantially not soluble in such a liquid medium, but rather forms a solid-in-liquid system.

In some embodiments, the dispersant is present at a dispersant-to-powder ratio ranging from about 2.5:1 to about 6:1 (v/w in ml/g), respectively, e.g., 2.5:1, 3:1, 3.5:1, 4:1, or 5:1, including any value and range therebetween.

By "around room temperature" it is meant to refer to at least one temperature value within the range of 10 °C to 40 °C, or 15 °C to 37 °C, e.g., 10 °C, 15 °C, 20 °C, 25 °C, 30 °C, 35 °C, 37 °C, or 40 °C, including any value therebetween.

The term "glycerol" means 1,2,3-propanetriol, and is also known as "glycerin", and may also meant to also encompass a derivative thereof. The term "derivative" refers to a compound containing essential elements of the parent substance.

As used herein, "thrombin" denotes an activated enzyme which results from the proteolytic cleavage of prothrombin (factor II). Thrombin may be produced by a variety of methods of production known in the art, and includes, but is not limited to, recombinant thrombin and plasma derived thrombin.

Human thrombin is a 295 amino acid protein composed of two polypeptide chains joined by a disulfide bond. Both human and non-human (e.g., bovine) thrombin may be used within the scope of the present disclosure.

The origin for thrombin used in the present disclosure may be from one or several sources including but not limited to: plasma (e.g., porcine plasma), recombinant bacteria and/or cells (see e.g., Vu et al., 2016, J. Viet. Env. 8(1):21-25), whole blood (pooled from several donations or not) and/or blood fraction (that may be pooled from several donations, e.g., plasma). Thrombin is available by manufacturers such as Johnson & Johnson, Baxter and CSL Behring either as a standalone product, e.g., EVITHROM^{®}, or as a component of a product e.g., EVICEL^{®}, TISEEL^{®}, Beriplast^{®}, and the like.

In exemplary embodiments, the powder comprises fibrinogen at a concentration of 12-20% w/w, fibrin at a concentration of 53-82% w/w, thrombin at a concentration of about 2-25% w/w and calcium. In some embodiments, the calcium is present at an amount of about 2% w/w. In some embodiments, the calcium is present at an amount of about 2% to about 4.5% w/w. In some embodiments, the calcium is present at an amount of about 2% to about 3% w/w. In some embodiments, the powder is in the form of porous particles. The term "porous" as used herein refers to a material which has voids throughout the internal structure.

In some embodiments, the thrombin is present at an amount of about 2000 to about 4000 IU per 1 gr powder. In some embodiments, the thrombin is present at an amount of about 3000 IU per 1 gr powder. In some embodiments, the thrombin is present at an amount of about 2000, 3000, or 4000 IU per 1 gr powder, including any value and range therebetween.

Herein "U" denotes a coagulation factor unit, that is a unit of a physiological measurement of a coagulation factor in 1 ml of human normal plasma.

"IU" refers to International Unit of a coagulation factor which is a physiological measurement in a given sample as compared to an adequate intentional standard, e.g., as determined by the clotting assay against an internal reference standard for potency concentration measurement that has been calibrated against, for example, the World Health Organization (WHO) Second International Standard for Thrombin, 01/580.

The term "fibrinogen" without more is intended to include any type of fibrinogen. "Fibrinogen" refers to monomeric and dimeric fibrinogen molecules having the monomer structure (AαBβγ), hybrid molecules, and variants thereof, whether naturally occurring, modified, or synthetic. The term "fibrinogen" may refer to fibrinogen from humans, but may include fibrinogen of any species, especially mammalian species, such as fibrinogen produced from porcine plasma.

The term "powder" refers to solid particulate material, typically, to dispersed dry solid particles, e.g., in the form of a plurality of particles of a solid characterized by small size of at least one dimension thereof, typically, within the range of from 0.1 to 1000 micrometers (µm), at times below 500 µm, and at times below 100 µm.

The term "solid" characterizes the state of the compound or composition at room temperature (e.g., 25°C) and at atmospheric pressure (760 mmHg), i.e. a compound or a composition of high consistency which retains its form during storage. This term in the present application also relates to non-fluid particles, or dissolved substance. As opposed to "liquid" compounds and compositions, the solid does not flow under its own weight. "Powdered" and "particulate" may be used interchangeably herein.

The term "paste" as used herein, relates to the consistency of the composition at at-least one temperature around the room temperature, and defines a fluid mixture of solid particles. Typically, paste has no fixed shape, and is therefore not a solid or a gas. The term "paste" according to the present disclosure may also include slurry, salve, and ointment. Slurry may functionally be regarded as a thin, oily paste. A paste according to the present disclosure may also include pores comprising an expandable gas, such as air. Accordingly, in some embodiments, the composition is a paste, or is in pasty consistency, at at-least one temperature at around the room temperature.

The term "aqueous solution" refers to a solution comprising water as the solvent. This term may include water, saline, or a bleeding site. In some embodiments, the aqueous medium is selected from water, saline, a calcium chloride solution or, in some embodiments, a buffered aqueous medium.

In exemplary embodiments, the aqueous solution comprises saline. As used herein, the term "saline" refers to water containing salt at a concentration greater than about 0.5% and less than about 20% by weight, e.g., about 0.9 w/w% sodium chloride solution in water, typically demineralized water.

In some embodiments, the particle size of at least 90% of the powder ranges from 10 to 800 µm. In some embodiments, the particle size of at least 90% of the powder ranges from 10 to 700 µm. In some embodiments, the particle size of at least 90% of the powder ranges from 100 to 800 µm. In some embodiments, the particle size of at least 90% of the powder ranges from 200 to 700 µm. In some embodiments, the particle size of at least 90% of the powder ranges from 100 to 600 µm. In some embodiments, the particle size of at least 90% of the powder ranges from 200 to 800 µm.

In some embodiments of any aspect of the present disclosure, the plurality of particles is characterized by a median particle size of about 100 to about 600 µm. In some embodiments of any aspect of the present disclosure, the plurality of particles is characterized by a median particle size of about 200 to about 500 µm.

The term "median", also referred to as "D50" in the context of particles size refers to the particle size that divides the population in half such that 50 % of the population is greater than or less than this size.

In some embodiments of any aspect of the present disclosure, the plurality of particles is characterized by a median particle size of less than about 500 µm.

In some embodiments, at least part of the powder is in the form of aggregate(s), agglomerate(s), or granulate(s). The term "aggregate" describes a particle formed from assembled components. Aggregates may optionally be made by a step of humidifying the powder composition; compacting, e.g., by roller and/or slugging the powder to form an aggregated structure; dehumidifying; milling; sieving the aggregates; and optionally dosing the resulting aggregates into a storage container or into a delivery device. The term "granulate" or "granulate material" may particularly denote a conglomeration of particles typically below 900 µm, below 800 µm, below 700 µm, or even below 500 µm. That is, at least part of the particles is not in the form of a discrete solid. In some embodiments, high shear mix is utilized to assemble particles of fibrinogen, thrombin, and optionally CaCl₂, into aggregates.

In some embodiments, at least part of the powder or the solid blend is in an aggregated or agglomerated form. The term "agglomerate" refers to a weak association of primary particles or aggregated particles usually held together by charge or polarity.

The term "at least part of the powder" refers to a part of the population of the particles in the powder e.g., at least between 10% to 100% of the powder, or at times, e.g., at least 25% of the powder. Thus, the term "at least part of the powder is in an aggregated form" refers to a part of the population of the particles in the powder e.g., at least between 10% and 100% forming an aggregate.

The term "flowable" also encompasses a viscous solution. The term "viscous solution" refers to a solution that has an increased resistance to flow, yet is capable of flowing. In some embodiments, the composition has viscosity greater than about 400 and is less than about 8000 Pa·s.

The term "viscosity" refers to a property of fluids and slurries that indicates their resistance to flow, defined as the ratio of shear stress to shear rate. Viscosity typically has a stated or an understood shear rate. Hereinthroughout, unless stated otherwise, in the context of viscosity the shear rate is 0.5 sec-1, and the viscosity is measured at ambient pressure and temperature conditions (referred to as "ambient conditions", i.e. around room temperature and atmospheric pressure). The term "shear rate" refers to the velocity gradient measured across the diameter of a fluid-flow channel, be it a pipe, annulus or other shape. Shear rate is the rate of change of velocity at which one layer of fluid passes over an adjacent layer.

In some embodiments, the composition has a viscosity greater than about 400 and is less than about 8000 Pa·s. In some embodiments, the composition has a viscosity ranging from about 1000 to about 7000 Pa·s. In some embodiments, the composition has a viscosity ranging from about 2000 to about 6000 Pa·s. In some embodiments, the composition has a viscosity ranging from about 3000 to about 5000 Pa·s. In some embodiments, the composition has a viscosity of about 2000 Pa·s, about 2500 Pa·s, about 3000 Pa·s, about 3500 Pa·s, about 4000 Pa·s, about 4500 Pa·s, about 5000 Pa·s, including any value and range therebetween.

In some embodiments, the composition has a uniform viscosity. The term "uniform viscosity", or homogenous viscosity", refers to a viscosity of at least about 3000 Pa·s and which varies within less than 50%, less than 40%, less than 30%, less than 20%, or less than 10%, by value of Pa·s.

In some embodiments, the composition has a stable viscosity. The term "stable viscosity" refers to a viscosity which varies within less than 50%, less than 40%, less than 30%, less than 20%, or less than 10%, by value of Pa·s, for at least 24 h at a temperature around the room temperature.

In some embodiments, the paste is characterized by a viscosity of about 2500 to about 4500, or 3000 to 4500 Pa·s, e.g., 2500, 2600, 2700, 2800, 2900, 3000, 3100, 3200, 3300, 3400, 3500, 3600, 3700, 3800, 3900, 4000, 4100, 4200, 4300, 4400 or 4500 Pa·s, including any value and range therebetween.

In some embodiments, the composition is in an injectable form. As used herein, the phrase "injectable form" refers to a flowable composition (e.g., may comprise crosslinked fibrinogen, polymerized fibrin and/or crosslinked fibrin in the form of particles small enough to allow for injection into a human subject, e.g., injection via a syringe and needle), as well as being of a suitable purity and non-toxicity for injection into a subject. The composition should be homogeneous and typically may have homogeneous rheological properties for passing smoothly while injected through needles of various internal diameters (e.g., 14 - 32 gauge). Thus, in some embodiments, the composition is capable of being passed in/through an applicator. In some embodiments, the composition is characterized by homogeneous viscosity, that is, the viscosity of the composition varies within less than |±40%|, less than |± 30%|, less than |±20%|, less than |±10%|, or less than |±5%|, within 4 h after adding the dispersant to the powder (see **Figure 3A****).**

As used herein, by "homogeneous" or "uniform", it is meant to refer to a uniform composition, viscosity, and texture throughout, typically, the powder is dispersed substantially evenly throughout the dispersant. Herein, by "dispersed substantially evenly" it is meant that the concentration of the powder within the dispersant of the composition varies within less than |± 30%|, less than |±20%|, less than |+10%|, less than |±5%|, by weight, throughout the dispersant, typically without a significant solid-liquid phase separation. In addition, the term "homogeneous" typically refers to a uniform appearing product (e.g., paste) which withstands a liquid/solid phase separation (e.g., less than 4% by weight) at 8 to 40°C, 10 to 30°C, or 20 to 30°C.

In some embodiments, the composition is characterized by a low swelling ratio. The term "swelling ratio" as used herein refers to the volume change in percentage of the paste upon immersing the paste in the liquid e.g., in blood, compared to the volume of the paste prior to immersing in the liquid, that is, the expansion extent of the paste forming between the state prior to adsorbing liquid (e.g., blood) and after adsorbing the maximal possible amount of liquid. Herein, "low swelling ratio" refers swelling ratio of less than about |±5%|.

Without being bound by any particular mechanism, it is assumed the low swelling ratio may allow achieving an improved hemostatic effect in small defect sites, in the absence of occupying effect, i.e., the paste is capable of staying in the bleeding site for a long time. Reference is made to **Figure 5A** in this regard.

In some embodiments, the composition is bioabsorbable. The term "bioabsorbable" refers to the ability of a tissue-compatible material to degrade in the body after implantation into non-toxic products which are eventually eliminated from the body or metabolized.

As used herein, and unless stated otherwise, the terms "weight ratio", "by weight", "w/w", "weight percent", or "wt. %", which are used herein interchangeably, describe the concentration of a particular substance out of the total weight of the corresponding mixture, solution, suspension, formulation or composition. As used herein, and unless stated otherwise, the terms "volume ratio", "by volume", "v/v", "volume percent", or "v%", which are used herein interchangeably describe the concentration of a particular substance out of the total volume of the corresponding mixture, dispersant, solution, suspension, formulation or composition.

The phase-component composition may be prepared by mixing a first component and a second component to make a suspension. As used herein, the term "phase-component composition" refers to a composition comprising at least two phases. Typically, but not exclusively, the two phases may be stored in separate containers and are mixed to create a mixture or suspension thereof before use, such as application of the mixture or the suspension to site in a subject (e.g., as shown in **Figure 1****).**

As used herein, the term "mixture" refers, but is not limited to, a combination of components in any physical form, e.g., blend, solution, suspension, dispersion, or the like.

The term "suspension", or any grammatical inflection thereof, as used herein, refers to a heterogeneous mixture of a solid in fine solute-like microparticles dispersed in a liquid or solvent-like phase. Typically, a suspension will have a tendency to settle, namely the fine particles of the solid matter may have the tendency to precipitate after a period of time, which, in some embodiments, is above 24 h. This period of time may depend on many factors, such as, and without being limited thereto, the substances of the microparticles and the liquid, the temperature, the pH, and other physical parameters like stirring and shaking, and the presence of other substances, such as dispersing agents, emulsifiers, surface-active agents, thickeners or the like.

In some embodiments, the composition further comprises fibrin. The term "fibrin" does not only refer to fully coagulated fibrinogen but further includes any mixture of fibrin and fibrinogen which may occur during formation of fibrin from fibrinogen using thrombin and, thus, includes any ratio of fibrinogen/fibrin and any grade of gelation and/or clotting conceivable as long as it has no negative impact on the final pasty texture of the composition. Moreover, the term "fibrin" further includes any partly or fully cross-linked, gelled or clotted form.

It is thus to be appreciated that fibrin per se exists as a polymeric, insoluble matrix comprising fibrils, each fibril comprising many fibrin molecules.

In some embodiments, the glycerol is present at a concentration of above about 30% by volume of the dispersant. In some embodiments, the glycerol is present at a concentration of above about 40%, by volume of the dispersant. In some embodiments, the glycerol is present at a concentration of up to about 80%, by volume of the dispersant. In some embodiments, the glycerol is present at a concentration of up to about 70%, by volume of the dispersant. In some embodiments, the glycerol is present at a concentration of up to about 60%, by volume of the dispersant.

In some embodiments, the glycerol is present at a concentration of above about 25% to about 80%, by volume of the dispersant. In some embodiments, the glycerol is present at a concentration of above about 35% to about 80%, by volume of the dispersant. In some embodiments, the glycerol is present at a concentration of above about 15% to about 60%, by volume of the dispersant. In some embodiments, the glycerol is present at a concentration of above about 30% to about 80%, by volume of the dispersant. In some embodiments, the glycerol is present at a concentration of above about 40% to about 60%, by volume of the dispersant.

In some embodiments, the glycerol is present at a concentration of about 25%, about 30%, about 35%, about 40%, about 45%, about 50%, about 55%, about 60%, about 65%, about 70%, about 75%, or about 80%, by volume of the dispersant, including any value and range therebetween.

In some embodiments, the dispersant has pH ranging from about 4.5 to about 7.

It was shown that samples of about 50% glycerol provided a pasty composition upon the dispersion in the above-mentioned dispersant, while providing both optimal viscosity and staying in place, as well as thrombin potency remaining, whereas more than samples of about 80% glycerol exhibited to high flowability resulted in composition being less capable of staying in place e.g., at a wound site. Further results showed that liquid-solid phase separation occurred for samples of less than 30% glycerol after 20 h.

In some embodiments, the composition is stable. The terms "stable", and "stability" when referring to the disclosed paste, mean that an active component within, e.g., thrombin at a certain temperature and after certain time duration remains at least 70 % active, that is, capable of forming a fibrin clot.

In some embodiments, the composition is stable. The term "storage stable" is to be understood as referring to the formulation, composition, typically being present in a container e.g., vial, or syringe that is stable under preselected storage conditions (e.g., pH), including pre-selected storage temperature, or pre-selected physical state of the composition. Stability can be determined by methods known in the art, e.g., by testing the absence of visible aggregations, remnant and/or undesired clots in the formulation under the pre-selected storage condition, for example, when the composition is in liquid or pasty form. Further, in accordance with the present disclosure, when referring to a stable sealant composition or formulation it is to be understood as one that, upon use, has an effective clotting time irrespective of some formulation's storage conditions, e.g., the sealant clots at essentially the same time period irrespective of whether it was stored at room temperature (e.g., 8 to 40°C) or at even lower temperatures (e.g., below 8°C).

In some embodiments, less than 25%, less than 24%, less than 23%, less than 22%, less than 21%, or less than 20%, by weight, remnant is detected after at least 20 h, at least 2 days, at least 3 days, at least 4 days, at least 5 days, at least 6 days, or at least 7 days at pH of 4 to 6.5 at about room temperature.

In some embodiments, the composition, in any embodiment thereof, further comprises an additional pharmaceutically active agent being contained within the composition or on a surface of the composition.

In some embodiments, the pharmaceutically active agent is selected from the group consisting of a therapeutically active agent and a labeling agent.

In some embodiments, the therapeutically active agent is selected from the group consisting of a stem cell, a growth factor, a bone morphogenetic protein, a cell, a cytokine, a hormone, a medicament, a mineral, a plasmid with therapeutic potential, and a combination thereof. As described herein, in some embodiments, the composition may further comprise at least one biologically active agent. Non-limiting biologically active agents that may be included in the composition include calcium, as well as therapeutic agents such as antibiotics, anti-inflammatory agents, growth factors, or clotting factors. Specifically, it is also possible that the disclosed composition comprises components which encourage the formation of the clot, such as, without being limited thereto, calcium salt, Factor VIII, Factor XIII, fibronectin, vitronectin, von Willebrand factor (vWF), which can be provided as a separate component or formulated with the blend or the paste.

The pharmaceutically active agent is optionally attached to the protein (i.e. fibrinogen or fibrin) matrix (e.g., covalently linked to the protein). The agent may be attached to the matrix after the matrix has been prepared and/or attached to the ingredients (e.g., protein and/or reducing sugar) which are then used to prepare the protein matrix, e.g., crosslinked fibrin.

Alternatively, or additionally, the agent is absorbed by the crosslinked protein matrix. Absorption may be obtained, for example, by contacting a crosslinked fibrinogen or fibrin matrix or a non-crosslinked fibrin matrix with a solution containing the agent (e.g., by dipping, soaking, washing), or by adding the agent prior to crosslinking (e.g., inclusion of the agent in the crosslinking solution, or inclusion of the agent in a solution of fibrinogen and thrombin).

Compositions described herein, as well as the contents of the abovementioned kits, may, if desired, be presented in a pack or dispenser device, which may contain one or more unit dosage forms containing the composition or ingredients (e.g., fibrinogen,) and/or reagents (e.g., dispersant) for preparing the composition. The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration (FDA) for prescription drugs or of an approved product insert. Compositions comprising a preparation of the invention formulated in a pharmaceutically acceptable carrier may also be prepared, placed in an appropriate container, and labeled for use for an indicated application and/or for treatment of an indicated condition, as further detailed herein. The term "preparation" refers to a composition being physiologically suitable for therapeutic use.

It will be appreciated that compositions of embodiments of the present disclosure may be attached to or included in medical devices, such as for promoting wound healing.

In another aspect of the present disclosure, the disclosed composition according to any aspect or embodiment thereof is for use in a method for preparing a fibrin sealant in/on an injured tissue of a subject, e.g., by applying the composition on a surface of the tissue.

In another aspect of the present disclosure, there is provided a method for preparing a fibrin sealant in/on an injured tissue of a subject, e.g., by applying the disclosed composition in any aspect and/or embodiment thereof as a hemostat on a surface of the tissue.

In some embodiments, the composition is substantially absent from oxidized cellulose. The term "oxidized cellulose" (or "OC") refers to a cellulose derivative in which at least some of the primary alcohol groups, e.g., on the carbon 6 of the anhydroglucose unit is oxidized to a carboxylic acid and is optionally functionalized. In some embodiments, the composition is substantially absent from oxidized regenerated cellulose (ORC).

In some embodiments, the composition is substantially absent from a buffering agent. In some such embodiments, a buffering agent refers to Tris, or Tris(hydroxymethyl)aminomethane buffer, or lysin. In some such embodiments, a buffering agent refers to Tris, or Tris(hydroxymethyl)aminomethane buffer, or lysin in a powder form.

The term "substantially absent" is defined herein to mean almost absent or completely absent. That is, it is meant to refer to less than 1%, less than 0.5%, less than 0.3%, less than 0.1% (w/w) of total weight of the composition, or 0% of total weight of the composition.

In another aspect of the present disclosure, there is provided a method of making the composition according to an embodiment thereof, e.g., a hemostatic composition characterized by viscosity ranging from about 3000 to about 5000 Pa·s at about 25°C and/or a swelling ratio of at most 10% at about 25°C, the method comprising the following steps being carried out sequentially:
(i) whipping a fibrinogen solution so as to obtain foam;
(ii) spraying a thrombin solution into the foam, so that the foam further incorporates the thrombin;
(iii) stirring the foam containing the thrombin so as to allow partial conversion of fibrinogen into fibrin;
(iv) lyophilizing the fibrin;
(v) milling the lyophilized fibrin, thereby obtaining a uniform powder, and
(vi) mixing the powder with a dispersant comprising an aqueous solution, glycerol and optionally thrombin, in a condition allowing making the hemostatic composition.

In some embodiments, the fibrinogen is in solution is at a concentration of least 20 mg/ml, e.g., 30 mg/ml, 40 mg/ml, 50 mg/ml, 60 mg/ml, 70 mg/ml, 80 mg/ml, 90 mg/ml, 100 mg/ml, including any value and range therebetween.

The term "whipping" as used herein, refers to the action of using a forward and rearward movement, whisk, or a mixer to beat a sample in order to rapidly incorporate air and produce foam.

As used in this specification, the term "foam" refers to a substance that is formed by trapping pockets of a gas in a liquid or solid. The term "spraying" is to be understood in a broad sense and may also involve terms such as sprinkling, atomizing, misting and the like. The term "milling" refers to a process whereby particles are reduced in size by impact with rapidly moving surfaces in a mill. In some embodiments, the mill is a ball mill, a bead mill, a jet mill or a disc mill. The term "mixing" refers to the blending of or stirring of the components (e.g. powder and dispersant) together.

In some embodiments, about 60 to about 95%, or, in some embodiments, about 70 to about 85%, by weight, of the fibrinogen is clotted (i.e. converted to fibrinogen).

The foam obtained in step (iii) is freeze dried (lyophilized) and stored for a later use, or may be freeze dried and then, right away be milled or ground into a powder. As used herein, the term "lyophilized" or any grammatical inflection thereof, refers to a product obtained by a process of freezing a solution and then reducing the concentration of water e.g., by sublimation to levels which do not support biological or chemical reactions, forming a "cake". Such a lyophilized "cake" having low protein density, may have a higher dissolution level. In order to dissolve the "cake" in the aqueous solvent, a solid dispersion can optionally be prepared by mechanically crushing and/or milling the "cake".

Typically, following lyophilization of the solution, a porous and spongy solid material, referred to as a "cake" (may also be referred to herein as "solid composition") is obtained.

Typically, the milling step can also be referred to as micronization. The term "micronization" is used when the particles that are produced e.g., from the solid composition, and are only a few up to several hundred micrometers in diameter. Micronization can be achieved by processes including, but not limited to, jet milling, pearl-ball milling, high-pressure homogenization, the RESS (Rapid Expansion of Supercritical Solutions) process, the SAS (Supercritical Anti-Solvent) method, or the PGSS (Particles from Gas Saturated Solutions) method. Typically, but not exclusively, micronization results in a 30 to 400- fold size reduction of the protein powder from its original size.

In some embodiments, the thrombin is present in the thrombin solution (in step ii) at a concentration of about 1500 to about IU/ml. In some embodiments, the thrombin is present in the thrombin solution is at a concentration of about 1550 to about 2000 IU/ml.

In some embodiments, the thrombin solution further comprises one or more additives such as, without being limited thereto, a calcium salt such as calcium chloride, and/or one or more excipients, e.g., selected from, without being limited thereto, one or more amino acids, albumin, saccharides, and/or saccharide derivatives.

The term "additive" is meant to be understood as any substance that can be added to a composition, and may also include an active additive such as a calcium salt as described herein.

Calcium is an important element in the clotting cascade, and may be needed for activation of factor XIII into factor XIIIa, which cross-links and stabilizes fibrin to generate an insoluble clot.

In some embodiments, the method further comprises adding a calcium salt to the powder obtained in step (v). In some embodiments, the calcium salt is CaCl₂, being at an amount of up to about 3%, by weight of the powder.

In some embodiments, an amount of calcium salt such as chloride is dissolved in the thrombin solution. In some embodiments, the amount of calcium chloride dissolved in the thrombin solution ranges from about 0.60 to about 3% w/v, or, in some embodiments, from about 0.65 to about 2.6% w/v.

The term "excipient" as used herein denotes a non-active or non-therapeutic agent added to a pharmaceutical composition e.g., to provide a desired consistency or stabilizing effect.

In some embodiments, the foam e.g., in the lyophilized form, is milled into powder with a median particle size below1000 µm. In some embodiments, the foam e.g., a lyophilized sample is milled into powder with a median particle size below 500 µm.

In some embodiments, the dispersant comprises about 30% to about 80% v/v glycerol in saline. In some embodiments, the dispersant comprises about 40% to about 80% v/v glycerol in saline. In some embodiments, the dispersant comprises about 40% to about 70% v/v glycerol in saline. In exemplary embodiments, the dispersant comprises about 50% glycerol in saline.

In some embodiments, the dispersant comprises thrombin. In some embodiments, the thrombin is added to the dispersant so as to catalyze blood clot upon use of the paste at the bleeding site. In some embodiments, the amount of thrombin in the dispersant ranges from about 100 to about 900 IU/ml. In some embodiments, the amount of thrombin in the dispersant ranges from 200 to 800 IU/ml. In some embodiments, the amount of thrombin in the dispersant ranges from 300 to 600 IU/ml. In exemplary embodiments, the amount of thrombin in the dispersant is about 400 IU/ml.

In some embodiments, the method is carried out in conditions selected from one or more from (a) to (e): (a) the thrombin is present at an amount of about 2000 to about 4000 IU per 1 gr; (b) the dispersant is present at a dispersant-to-powder ratio ranging from 3:1 to 5:1 (v/w; ml/g), respectively; (c) at least about 70% of the foam obtained in step (ii) comprises fibrinogen; (d) the method is carried out at a temperature in the range of from 0 to 30°C; and (e) the pH of the dispersant is in the range of from 4.5 to 7. In some embodiments, the conditions are selected from one or more from (a) to (c).

In some embodiments, such conditions allow at least partial conversion of fibrinogen into fibrin.

According to another aspect, the disclosed composition according to any embodiment thereof is for use in a method for treating a bleeding tissue, the method comprising applying the composition to the bleeding tissue, by obtaining a sealant at the site upon application. Accordingly, there is provided a method of controlling bleeding comprising applying the herein disclosed composition to a bleeding site.

In the context of the present invention, the term "sealant", also referred to as "fibrin sealant", and "biological glue", is to be understood as an adhesive, glue, or hemostat, e.g., such as one originates or being derived from the disclosed composition, having ingredients that upon contact with, or in proximity to, a tissue and/or blood, reacts to subsequently form a clot, and may further act as a tissue adhesive, and thereby prevents, reduces, or stops bleeding, joins structures and/or seals physiological leaks, e.g., of cerebrospinal fluids (CSF), lymph, bile, gastrointestinal (GI) content, air leak from lungs etc. In some embodiments, the sealant formulation also comprises one or more therapeutics such that upon natural degradation of the clot formed in the body, the therapeutic is released. The therapeutic may be, without being limited thereto, drug(s), such as antibiotics, analgesics, anti-inflammatory drugs, cancer drugs etc., cells including, for example, any type of stem cells e.g., Embryonic Stem (ES) cells, adult stem cells, Induced Pluripotent Stem Cells (iPSCs) etc., from human or other origin.

The term "hemostatic" refers to an ability to prevent, reduce, or stop blood loss e.g., from wounds, such as surgical or traumatic wounds, e.g., by promoting blood clot formation or assist in hemostasis. "Hemostasis" (or "haemostasis") refers to the first stage of wound healing. It is a process which causes bleeding to stop. By "assist in hemostasis" it is meant to help reduce or stop bleeding. By "applied to a bleeding tissue" it is meant to refer to e.g., a topical application of the composition at the bleeding site, e.g., at a surgical site to control bleeding. Control of bleeding is needed in various situations including treatment of wounds.

As used herein, the term "bleeding" refers to extravasation of blood from any component of the circulatory system. A "bleeding" thus encompasses unwanted, uncontrolled and often excessive bleeding in connection with surgery, trauma, or other forms of tissue damage, as well as unwanted bleedings in patients having bleeding disorders. The term "blood", or any grammatical inflection thereof, also includes blood fractions, such as plasma.

The term "trauma" is defined as an injury caused by a physical force; non-limiting examples include the consequences of vehicle accidents, gunshots and burns.

As used herein, the terms "controlling", "preventing", or "reducing", which may be used herein interchangeably in the context of the bleeding, indicate that the rate of the blood extravagated is essentially nullified or is reduced e.g., by 10%, at least 20%, at least 30%, at least 40%, at least 50%, at least 60%, at least 70%, at least 80%, at least 90%, or even by 100%, of the initial rate of bleeding, compared to situation lacking the contact of the disclosed composition in/on the bleeding site. Methods for determining the level of appearance of bleeding are known in the art.

Further, the terms "controlling", "preventing" or "reducing", in the context of the bleeding may also encompass at least partially sealing blood vessels at the bleeding site e.g., in soft tissues.

As used herein, the term "subject" shall mean any animal including, without limitation, a human, a mouse, a rat, a rabbit, a non-human primate, or any other mammal. In some embodiments, the subject is human, e.g., a human patient. The subject may be male or female.

As used herein, the term "injured tissue" (or "damaged tissue") refers to disruption of the normal continuity of structures caused by a physical (e.g., mechanical) force such as in incisions caused by surgery, a biological (e.g., thermic or actinic force), or any chemical means. The term "injured tissue" also encompasses contused tissues, stabbed, or lacerated tissue, as well as open, puncture, and injuries caused e.g., by ripping, scratching, pressure, and biting. The term "injured tissue" also encompasses wounds and bleeding sites as described hereinthroughout.

The term "tissue" may refer to a tissue selected from a soft tissue and a bone tissue.

The term "soft tissues" as used herein relates to body tissue that is not hardened or calcified. This term especially relates to soft tissues that are vascularized and therefore may be a source of bleeding. Examples for such tissues include, but are not limited to, connective tissue (such as tendons, ligaments, fascia, skin, fibrous tissues, fat, and synovial membranes), muscles, and internal organs. In general, soft tissues are meant to exclude bone tissues.

Thus, in some embodiments, the disclosed composition in any embodiment thereof may be used applied in difficult-to-reach bleeding sites, for example during MIS such as, e.g., endoscopy. One of the common forms of endoscopy is laparoscopy which is minimally invasive inspection and surgery inside the abdominal cavities.

The term "surgery" also encompasses the time during or after surgical or diagnostical procedures. Further non-limiting examples of procedures include neurosurgery, abdominal surgery, cardiovascular surgery, thoracic surgery, head and neck surgery, pelvic surgery and skin and subcutaneous tissue procedures. For at least one of these situations, the composition of the invention may serve as a suitable sealant, which may permit adhesion to the bleeding wound, and thus addresses hemostasis without being dependent on the condition of the wounded tissue, e.g., severity of bleeding. The formation of the sealant occurs in a relatively short period of clotting time subsequent to applying the disclosed composition, according to any aspect and/or embodiment thereof, on an injured tissue. Typically, using after 1 to 3 min tamponade, an incomplete clot may form which enables to stop bleeding, but more time may be needed for clotting reacting up to the paste surface thus forming a complete clot.

In another aspect of the present invention, there is provided a method of treating a wound comprising the step of applying (e.g., contacting) the disclosed composition in any aspect and/or embodiment thereof onto and/or into the wound of a subject in a need thereof.

By "treating a wound" it is meant to encompass, inter alia, reducing blood loss at a bleeding site of a tissue (in vivo), e.g., in a patient undergoing surgery. Reference is made to **Figure 6B****,** showing a high hemostatic success rate (above 75%) of the tested samples disclosed herein.

Accordingly, in some embodiments, the method is for reducing blood loss at a bleeding site of a tissue and/or making a sealant layer in/on such a tissue, e.g., in a patient undergoing surgery, the method comprising contacting the disclosed composition in an embodiment thereof with the bleeding site and/or its proximity. Optionally, the method comprises first applying on such a tissue an aqueous solution, such as saline, and thereafter applying on the aqueous solution the disclosed composition, so as to promote a fast clotting-time. Accordingly, in some such embodiments, the composition is for use as a hemostat in a bleeding site of a tissue.

According to another aspect of embodiments of the present invention, a kit is provided for generating a composition described herein. It is appreciated that the consistency of the composition is such that it can be applied, for example, by spreading or by sticking the composition directly onto the bleeding site. Accordingly, the composition does not need to be further spread on or applied to a solid surface, object, or other solid medium such as a strip or a film in order to be in the appropriate form for applying onto the bleeding site. Nevertheless, a suitable applicator, such as, for example, a syringe, may be used in order to apply, spread or stick the composition onto the bleeding site, for the purpose of easy access and handling, see **Figure 1****.**

In an additional aspect and/or embodiment, the present invention provides a kit comprising: a) a container containing the composition of the invention as described above, b) an applicator for applying the composition to a tissue, and c) optionally instructions for use.

In another embodiment, the present invention further provides a hemostatic kit comprising a container containing the herein disclosed composition in an embodiment thereof. In another embodiment, the present invention provides a hemostatic kit comprising a container containing a powder comprising fibrinogen or fibrin and thrombin, wherein the fibrinogen or the fibrin is present at an amount of at least about 70% by weight; and (b) a container containing a dispersant comprising glycerol and optionally an aqueous solution, and optionally (c) instructions for use.

In some embodiments, the kit further comprises an applicator device.

In some embodiments, the powder comprising fibrin and thrombin is obtained by a method described herein in an embodiment thereof.
In some embodiments, the dispersant comprises a thrombin powder. Alternatively, the kit comprises a thrombin powder or a thrombin solution in a separate container e.g., in a vial. The thrombin powder may be at an amount ranging from 200 to 600 IU e.g., about 400 IU per mg or per ml.

According to some embodiments, there is provided a kit for preparing a composition comprising (i) a powder comprising fibrinogen, fibrin and optionally thrombin, and optionally (ii) a dispersant comprising glycerol and optionally an aqueous solution. Upon preparation, the composition is in the form of a pasty suspension at a room temperature. Each of (i) and (ii) may be present in a separate container.

In some embodiments, the powder comprises at least 60% fibrin, or, in some embodiments at least 70% fibrin, by weight.

Any aspect and embodiment of the hereinthroughout disclosed composition, may be incorporated into the aspect and embodiments of the kit, as well as embodiments of the dispersant, and/or the powder.

Optionally, the fibrinogen or fibrin, and thrombin are each packaged individually (e.g., in dry form or in solution) in the kit. Thus, each of the two ingredients is packaged in separate packaging material, in addition to the packaging material of the whole kit. In some embodiments, the fibrinogen or fibrin, and thrombin are packaged together (e.g., as a dry powder) in the kit in the same packaging material, and optionally the dispersant is provided in an additional container.

In some embodiments, the kit further comprises instructions on how to combine the ingredients of the kit and/or how to combine the ingredients of the kit with an additional ingredient (e.g., a dispersant), in order to produce the desired composition.

Optionally, the kit further comprises a dispersant. A dispersant such as glycerol may be in pure form or in a solution with another liquid (e.g., water, saline or aqueous buffer). Optionally, the dispersant is packaged individually, apart from the powder. Alternatively, the dispersant is packaged in combination with the powder, for example, as a ready-for-use composition described herein. In such embodiments, the kit may further contain a measuring means, e.g., a measuring cylinder, to measure the volume of the dispersant or a component thereof.

Additionally, or alternatively, the hemostatic kit may comprise a syringe containing the blend, mixture or powder and another syringe containing the dispersant. For example, dual-syringe mixing devices may produce a substantially homogenous paste mixture by combining initially separate liquid and powder(s) and then passing the blended contents back and forth between two connected syringes via interconnected outlet(s). Therefore, a low expression force for dispensing the paste from a syringe may be preferred for ease of mixing and ultimately for deployment of the resulting paste. The desired expression force may be less than 1.51 lbf.

In some embodiments, at least one of the containers in the kit is a pre-filled syringe. In some embodiments, a syringe is provided in addition to the container(s) of the kit. In some embodiments, the container is in a specific type, such as a vial or an applicator such as syringe.

In some embodiments, at least one of the containers in the kit is a pre-filled syringe. In some embodiments, a syringe is provided in addition to the container(s) of the kit. The term "container" may refer to any generic structure such as a vessel or a vial, that may contain the powder, the dispersant as disclosed hereinthroughout, or any component thereof, or the paste.

The kit may be applied using an applicator device which may be used for administering several and sequential injections of the composition. In one embodiment, the applicator device enables multiple injections of a fixed-dose of the mixed components on a 2-D surface of a tissue while moving the device. In one embodiment, the applicator has a syringe and a tip or a needle configured to be connected to the applicator. In one embodiment, the applicator has a syringe with an injection needle, which is optionally automatically retracted from the patient's skin after the injection is completed without the need for the administrator to lift the device upward from the injection surface. In one embodiment, the kit may be used for the administration of a sealant.

The hemostatic kit of the invention may be a kit for use in reducing, preventing or stopping blood flow, e.g., in open wounds, and it may be used for reducing, preventing or stopping blood flow during a procedure, including before, or after a surgical procedure such as, for example, laparoscopic surgery, neurosurgery, abdominal surgery, cardiovascular surgery, thoracic surgery, head and neck surgery, pelvic surgery and skin and subcutaneous tissue procedures. The kit may be used for reducing or preventing blood flow from the skin, or in internal organs.

In one embodiment, this kit may be stored at room temperature, such as at a temperature in the range of 8 to 40°C, or at lower temperatures.

In some embodiments of any aspect of the kit or the composition disclosed herein, the powder or the blend may comprise an additive e.g., calcium salt and/or one or more excipients, e.g., selected from, without being limited thereto, one or more amino acids, albumin, saccharides, and/or saccharide derivatives.

In some embodiments of the kit or of the composition, the composition comprises calcium salt, e.g., calcium chloride (CaCl₂) e.g., at a concentration of 0.5% to about 4%, or 2% to 4%, or 2.5% to 3.5%, by weight of the powder. In some embodiments, the calcium salt, e.g., calcium chloride is present at a concentration of 0.5%, 1%, 1.5%, 2%, 2.5%, 3%, 3.5%, or 4%, by weight of the blend, including any value and range therebetween. In some embodiments, the calcium salt is present in a thrombin solution.

In some embodiments, the fibrinogen is present at a concentration range of 65% to 80%, 65% to 95%, or 70% to 90%, by weight of the powder. In some embodiments, the fibrinogen is present at a concentration of 65%, 70%, 75%, 80%, 85% or 90%, by weight of the powder, including any value and range therebetween. In some embodiments, the fibrinogen is present at a concentration of about 80% by weight of the powder. In some embodiments, the fibrin is present at a concentration range of 65% to 80%, 65% to 95%, or 70% to 90%, by weight of the powder. In some embodiments, the fibrin is present at a concentration of 65%, 70%, 75%, 80%, 85% or 90%, by weight of the powder, including any value and range therebetween. In some embodiments, the fibrin is present at a concentration of about 80% by weight of the powder.

In exemplary embodiments, the blend or the powder comprises about 70-80 % fibrinogen or fibrin powder, by total weight of the powder, by weight; 2000-4000 IU thrombin/dose powder. Herein "dose" means about 1.2 g.

In some embodiments of any aspect of the kit and/or compositions, the disclosed composition may be used in conjunction with a backing, pad, bandage, gauze, sponge, scaffold, or matrix to provide mechanical strength to cover the wound surface. In this case, the instant paste is supported on a pad for ease of application or tamponade.

In some embodiments of any aspect of the kit and/or compositions, the composition is sterile. Especially when handling blood products, the sterility issue is crucial, and specifically the issue of viral inactivation. In general, viral inactivation may be carried out by any number of methods, including solvent detergent, heat inactivation, irradiation, and nanofiltration. Typically, the standard for viral inactivation requires using two different methods. Additionally, FDA standard for sterility requires filtration.

The term "sterile" as used herein means having a low bioburden, effectively being germ-free, e.g., essentially or even absolutely being free from microorganisms, e.g., bacteria and viruses. Sterilization is the process of reducing the bioburden to an effectively germ-free level. A sterile liquid, powder or paste is generally defined as a liquid, powder or paste that has underwent sterile filtration.

### General

The terms "comprises", "comprising", "includes", "including", "having", and their conjugates mean "including but not limited to". The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

In those instances where a convention analogous to "at least one of A, B, and C, etc." is used, in general such a construction is intended in the sense one having skill in the art would understand the convention (e.g., "a composition having at least one of A, B, and C" would include but not be limited to compositions that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B".

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

The term "about" as used herein means that values that are 10% above or below the indicated value are also intended to be included. Unless otherwise indicated, all numbers such as, without limitation, those expressing, for example, ratios, weight, mole/mole, amounts, viscosity, temperatures, etc., are to be understood as being modified in all instances by the term "about". Accordingly, unless indicated to the contrary, the numerical parameters set forth in this description and attached claims are approximations that may vary by up to ±10% depending upon the desired properties sought to be obtained by the present invention.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

### EXAMPLE 1: FLOWABLE COMPOSITION PREPARATION

The object of this study is to develop a flowable hemostat with both flowability and adherence to tissue after applying it into bleeding sites during a surgery.

### Materials and Methods

**Table 1 - Materials**

| **No.** | **Sample Information** | **Note** |
|---|---|---|
| 1 | Fibrinogen solution | The major component is fibrinogen |
| 2 | Thrombin solution | The major component is thrombin |
| 3 | Fibrinogen powder* | Fibrinogen solution is dried into powder through spraying |
| 4 | Thrombin powder | Thrombin solution is dried into powder through spraying |
| 5 | OC/ORC powder* | Oxidized Regenerated Cellulose (ORC) fiber was processed into powder |
| 6 | Lysine/Tris* | In the form of a powder, as buffering agent is selected from Tris, lysine, or a combination thereof. |
| 7 | CaCl₂ | In the form of a powder |
| 8 | Glycerol in saline | As binder in granulation and dispersant for flowable paste |

| | | |
|---|---|---|
| * For the preparation of the "ORC Paste", see below. | | |

The source of fibrinogen and thrombin was porcine blood plasma, which was fractionated to obtain fibrinogen and thrombin, and supplied by Bioseal Biotech Co. Ltd, located in Guangzhou, China.

### Fibrinogen Sealant Powder Preparation

The fibrinogen/fibrin sealant powder (FSP) was obtained by lyophilizing and milling fibrin foam. This foam was produced by mixing porcine plasma extracted fibrinogen and thrombin in advance as follows: first, fibrinogen solution (at least 20 mg/ml) was whipped to fully foam (the foam should not slide off when scooped up and turned over). The foaming was carried out to help to create the porous structure with the proteins, such that the cross-linking reaction can occur in a larger space, resulting in a uniform and efficient cross-linking.

Next, a small amount of CaCl₂ (0.65-2.6% w/v) was thoroughly dissolved in the thrombin solution (1555-2000 IU/ml) and mixed well. Next, the thrombin solution (with the CaCl₂) was sprayed, using a spray tip attached to a syringe applicator, onto the foam, following by stirring, to allow cross-linking. It is estimated that about 70-85% of the fibrinogen was clotted during the process.

Next, a lyophilization step was carried out. After lyophilization, the water inside of the porous structure could be easily evaporated, and the remaining porous structure (the "cake") was easily and uniformly milled for the preparation of a homogenous flowable paste. The lyophilized samples were milled into powder with particle size < 500 µm. It is noteworthy, without being bound by a mechanism, that lyophilization helped to maintain the activity of unreacted fibrinogen (around 20 to 30% of the fibrinogen remained unreacted) which could be utilized to form the in-situ sealant upon applying into bleeding site. The parameters of the lyophilization were: pre-freezing at -45°C for 2 h; primary drying at 15°C for 96 h; secondary drying at 37°C for 24 h (using SCIENTZ Dry Vacuum Pump Freeze Dryer, SCIENTZ-50F/B Type, Ningbo Scientz Freeze-Drying Equipment Co., Ltd)

The weight ranges of each component in the obtained powder were: fibrinogen 12-20% w/w, fibrin 53-82% w/w, thrombin 4-25% w/w, CaCl₂ about 2% w/w. or 2.3% w/w in case of CaCl₂·H₂O and 2.6% w/w for CaCl₂·2H₂O.

### Paste preparation

The paste was obtained by mixing the powder (1 g) with a dispersant (3 to 5 ml, about 50% v/v glycerol in saline) with thrombin powder (3300 IU potency with about 21 mg a CaCl₂). The dispersant should have viscosity high enough for being capable of staying at a bleeding site for a certain time. It was found that using a low concentration of glycerol (below 30% by volume) in the dispersant had resulted in a phase separation of the glycerol and water. On the other hand, too high concentration (above 70%) resulted in a too viscous paste. The dispersant had pH of about 4.5 to 7.

The thrombin (400 IU/ml) was added also with the dispersant to make sure there was enough amount of thrombin to catalyze blood clot upon use of the paste at the bleeding site.

**Figures 2A-B** present a flowchart with photographic images showing an exemplary embodiment of process making the powder of the invention (Figure 2A), and a photographic image of the obtained paste (Figure 2B).

### Comparative Formulation

Comparative fibrinogen and thrombin formulation ("ORC paste") was also made from porcine fibrinogen and thrombin solution. The solution was processed into powder by spraying, and combination with ORC, lysine, and calcium salt. ORC powder was prepared by grinding or milling the corresponding fabric fibers to obtain powder without carrying out a step of foaming. The weight ratio of the ORC to fibrinogen was between from 1:1 to 1:2. The ORC paste comprised a buffering agent e.g., in the form of a powder (Tris, lysine, or a combination thereof). Data for the ORC Paste components: ORC: 0.6 g; fibrinogen: 0.3 g; thrombin: 3300 IU in one syringe paste (around 5ml); lysine: 0.2 g; CaCl₂: 0.02 g; dispersant: 50% glycerol in saline, by volume. The ORC paste was prepared without carrying out foaming and lyophilization steps. The lysine was added to reduce the acidity of the ORC. Such acidity might denature plasma proteins (e.g., thrombin).

### EXAMPLE 2: FIBRIN SEALANT PASTE CHARACTERIZATION

### Viscosity

FS Paste viscosity test was carried out using an oscillatory rheometer (Anton Paar Rheometer) of parallel plate geometry of 25 mm diameter with 1.25 mm gap. The results are shown in **Figures 3A-B.** It can be seen that the fibrin paste after reconstitution has a stable and uniform viscosity at room temperature for at least 24 hours. It can also be seen that the FS paste was more stable compared with the paste prepared with the ORC and with a higher viscosity, allowing the FS paste to stay at wound site for a longer time.

### Swelling Ratio

In exemplary procedures, the FS paste swelling ratio was tested by adding fibrinogen (70mg/mL, 2ml) to the paste (1g FS powder + 4.5 ml dispersant) and then incubating it in 37°C for 30 min, obtaining a solidified paste. The solidified paste was cut into ~1.0 g sections. After weighing, the sections were incubated in phosphate-buffered saline (PBS) for 24 h, allowing the sections to swell, see Figure 4A, following calculating the swelling ratio by weight increase. The results are shown in Figure **4B****.** A comparative test was made with a gelatin matrix, using SURGIFLO^{™} Haemostatic Matrix with Thrombin as the paste with the fibrinogen added. The results show that the FS paste of the invention has a low swelling rate compared to the gelatin sample. Due to the low swelling rate, the FS paste has no "occupying effect", and can stay in the bleeding site for a longer time, providing an improved hemostatic effect.

### In-vitro Clotting Time

In exemplary procedures, an amount of 0.5 g FS paste was placed in a glass vial and then an amount of 4 mL pig blood anticoagulant (sodium citrate, 3.2-3.8% w/v) was added. The vial was gently shaken or vortexed for a couple of seconds to allow contact between the blood and the paste to mix FS paste with the blood. Next, the vial was tilted to observe blood clotting. In case of complete clotting, the clotted blood stays in the upper part of the vial once being tilted or turned upside down. In case of incomplete clotting, blood remains fluid and drains towards the lower part of the vial due to the gravitational force. The time when the blood was no longer flowing was recorded. The results demonstrated a clotting time of 14 to 16 seconds, i.e. less than 20 seconds, indicating a good efficacy of blood coagulation in vitro.

Tested samples taken from the vials demonstrate that the flowable paste of the invention easily and uniformly absorbs blood (Figure 5A), as compared to the ORC paste (Figure 5B), thereby, having an improved potential to accelerate the coagulation cascades and to form endogenous fibrin clot in vivo.

### Animal Study- Efficacy Test in In-vivo Spleen Punch Model

In exemplary procedures, a wound defect, approximately 6 mm in diameter and 3 mm in depth, was made using a modified 6 mm biopsy punch (placing an elastic band as a 3 mm depth stop around the circumference of the biopsy punch). The center core (tissue plug) was removed with surgical forceps and scissors. One new site was made for each test or control article being tested, and similar sized defects were made for each treatment group. A sample test was added to the wound site (having moderate bleeding) and was applied to cover the wound site, and the sample was then pressed in wound with wet gauze for 2 minutes. Next, the gauze was gently removed 2 minutes later, and it was observed whether the wound kept bleeding; see Figure 6A. If no activity bleeding was found, the hemostasis was successful; otherwise, it failed. Negative controls were wet gauze press, and the ORC flowable paste. The success rate of hemostasis is the percentage of the number of successful hemostatic divided by the total number of sample test. The results are shown in **Figure 6B****,** presenting the hemostatic success ratio of the FS paste. As shown in **Figure 6B** the FS paste exhibited a hemostatic success of 80% compared to that of 40% of the ORC paste.

Taken together, comparing with ORC-Flowable, this invention strengthened the hemostasis efficacy through improving the formula with extra FSP (fibrin sealant powder) which helped to enhance blood absorption. Also, and without being bound by a particular mechanism, the porous powder in flowable paste allows blood to be absorbed easily and exposed to high concentration of thrombin, thereby accelerating the coagulation cascades to form endogenous fibrin clot. Also, without being bound by a particular mechanism, this porous structure remains in the powder at least partially (20 to 30% of the pores) after milling the porous cake, providing more voids and contact with the bleeding site, thereby improving the hemostatic effect. This flowable hemostatic composition can be applied to difficult-to-access bleeding sites and achieve hemostasis. **Figure 7A** presents the porous structure of the powder of the invention compared to the structure of the powder in the ORC paste **(****Figure 7B****).** The estimated pores size of the powder of the invention ranges from about 10 to 30 µm.

Although the invention has been described in conjunction with specific embodiment thereof, it is evident that many alternatives, modifications and variations will be apparent to those skilled in the art. Accordingly, it is intended to embrace all such alternatives, modifications and variations that fall within the spirit and broad scope of the appended claims.

## Claims

1. A two-phase flowable composition in the form of a paste a room temperature comprising:
(iii) a powder comprising fibrinogen and fibrin, being present together at an amount of at least about 70% by weight; thrombin; and
(iv) a dispersant comprising glycerol and an aqueous solution,
wherein:
the dispersant is present at a dispersant-to-powder ratio ranging from about 3:1 to about 5:1 (v/w; ml per g), respectively; and wherein:
the glycerol is present at a concentration of above about 30% by volume of said dispersant.

2. The composition of claim 1, comprising fibrin at an amount of about 60% to 80%, by weight.

3. The composition of claim 1 or 2, wherein the fibrin is at least partially cross-linked.

4. The composition of any one of claims 1 to 3, wherein the fibrinogen and/or fibrin are present in the powder at an amount of about 80% by weight.

5. The composition of any one of claims 1 to 4, wherein the powder further comprises a calcium salt.

6. The composition of claim 5, wherein the calcium salt is CaCl₂ or a hydrate thereof, being at an amount of up to about 3%, by weight.

7. The composition of any one of claims 1 to 6, wherein the thrombin is present at an amount of about 2000 to about 4000 IU per 1 gr powder.

8. The composition of any one of claims 1 to 7, wherein at least 80% of the powder is in the form of particles sized below 500 µm.

9. The composition of any one of claims 1 to 8, wherein at least part of the particles are porous.

10. The composition of any one of claims 1 to 9, wherein the glycerol is present at a concentration ranging from about 30% to about 80%, by weight of the dispersant.

11. The composition of any one of claims 1 to 10, wherein the aqueous solution comprises saline.

12. The composition of any one of claims 1 to 11, being **characterized by** uniform viscosity.

13. The composition of any one of claims 1 to 12, being **characterized by** viscosity ranging from about 3000 to about 5000 Pa·s at about 25 °C*.*

14. The composition of claim 13, wherein said viscosity varies within less than about 30% after storing at about 25 °C for 24 h.

15. The composition of any one of claims 1 to 14, being **characterized by** a swelling ratio of at most 10% at about 25 °C upon contact with blood.

16. The composition of any one of claims 1 to 15, being absent from oxidized cellulose.

17. The composition of any one of claims 1 to 16, being absent from a buffering agent.

18. The composition of any one of claims 1 to 17, being a hemostatic composition.

19. The composition of any one of claims 1 to 18, for use in a method for treating a bleeding tissue, the method comprising applying said composition to the bleeding tissue.

20. The composition of any one of claims 1 to 19, wherein the method comprises minimal invasive surgery e.g., laparoscopy.

21. A method of making a hemostatic composition **characterized by** viscosity ranging from about 3000 to about 5000 Pa·s at about 25 °C and/or a swelling ratio of at most 10% at about 25 °C, the method comprising the following steps performed subsequently:
(vii) whipping a fibrinogen solution so as to obtain foam;
(viii) spraying a thrombin solution into said foam, so that the foam further incorporates said thrombin;
(ix) stirring the foam containing the thrombin so as to allow at least partial conversion of fibrinogen into fibrin;
(x) lyophilizing said fibrin;
(xi) milling said lyophilized fibrin, thereby obtaining a uniform powder, and
(xii) mixing said powder with a dispersant comprising an aqueous solution, glycerol and thrombin, wherein the method is made in a condition allowing making said hemostatic composition.

22. The method of claim 21, wherein the condition is selected from one or more from (a) to (c):
(a) the dispersant comprises thrombin an amount of about 200 to about 800 IU per 1 ml;
(b) the dispersant is added to the powder at a dispersant-to-powder ratio ranging from 3:1 to 5:1 (v/w; ml/g), respectively, and
(c) at least about 70% of the foam obtained in step (ii) comprises fibrin.

23. The method of any one of claims 21 or 22, wherein the thrombin solution in step (ii) further comprises a calcium salt.

24. The method of claim 23, wherein the calcium salt is CaCl₂, being at a concentration of 0.5 to about 3% w/v.

25. A kit comprising:
(a) a container containing a powder comprising, fibrinogen, fibrin, and thrombin, wherein the fibrinogen and fibrin are present at a total amount of at least about 70% by weight; and
(b) a container containing a dispersant comprising glycerol and optionally an aqueous solution, and optionally
(c) instructions for use.

26. The kit of claim 25, further comprising an applicator device.
